(19) Europäisches Patentamt / European Patent Office / Office européen des brevets

(11) **EP 2 601 520 B1**

(12) **EUROPEAN PATENT SPECIFICATION**

(45) Date of publication and mention of the grant of the patent:
**14.05.2014 Bulletin 2014/20**

(21) Application number: **11746001.4**

(22) Date of filing: **01.08.2011**

(51) Int Cl.:
***G01N 33/487*** (2006.01)

(86) International application number:
**PCT/GB2011/001157**

(87) International publication number:
**WO 2012/017198 (09.02.2012 Gazette 2012/06)**

(54) **METHOD FOR IMPROVED ACCURACY FOR TEMPERATURE CORRECTION OF GLUCOSE RESULTS FOR CONTROL SOLUTION**

SYSTEME UND VERFAHREN FÜR ERHÖHTE GENAUIGKEIT BEI DER TEMPERATURKORREKTUR VON GLUKOSEERGEBNISSEN FÜR EINE KONTROLLÖSUNG

SYSTÈMES ET PROCÉDÉS PERMETTANT D'OBTENIR UNE PRÉCISION ACCRUE DE CORRECTION DE LA TEMPÉRATURE DE RÉSULTATS DE GLUCOSE POUR UNE SOLUTION TÉMOIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.08.2010 US 370211 P**
**02.08.2010 US 370069 P**

(43) Date of publication of application:
**12.06.2013 Bulletin 2013/24**

(73) Proprietor: **Cilag GmbH International**
**6300 Zug (CH)**

(72) Inventors:
• **CRAGGS, Adam**
  **Inverness IV2 6RH (GB)**
• **MACKINTOSH, Stephen**
  **Inverness IV3 8QE (GB)**
• **RODGERS, Jamie**
  **Inverness IV2 4LL (GB)**

(74) Representative: **Brunner, John Michael Owen**
**Carpmaels & Ransford LLP**
**One Southampton Row**
**London WC1B 5HA (GB)**

(56) References cited:
**EP-A2- 2 138 841**

**Description**

BACKGROUND

Analyte concentration determination in physiological fluids (e.g., blood or blood derived products such as plasma) is of ever increasing importance in today's society. Such assays find use in a variety of applications and settings, including clinical laboratory testing, home testing, etc., where the results of such testing play a prominent role in the diagnosis and management of a variety of disease conditions. Analytes of interest include glucose for diabetes management, cholesterol for monitoring cardiovascular conditions, and the like.

A common method for analyte concentration determination assays is based on electrochemistry. In such methods, an aqueous liquid sample is placed into a sample reaction chamber in a sensor, e.g., an electrochemical cell made up of at least two electrodes, i.e., a working electrode and a counter electrode, where the electrodes have an impedance that renders them suitable for amperometric or coulometric measurement. The component to be analyzed is allowed to react with a reagent to form an oxidizable (or reducible) substance in an amount proportional to the analyte concentration. The quantity of the oxidizable (or reducible) substance present is then estimated electrochemically and related to the analyte concentration in the sample.

SUMMARY

Applicants have discovered that certain existing temperature correction for determining the glucose concentration could be improved to have greater accuracy. The electrochemical cell can be in used in various sample analyzing devices such as glucose sensors or immunosensors. The analyzed sample can include blood. The blood can include whole blood. The analyte for which the concentration is being analyzed can include glucose. The assaying of a glucose concentration may include a physical transformation of glucose into gluconic acid. The enzyme GDH with the flavin adenine dinucleotide (FAD) co-factor may be used to catalyze the transformation of glucose into gluconic acid. The analyte for which the concentration is being analyzed can include C-reactive protein. The method according to the invention is concerned with determining a concentration of glucose in a control solution sample, the method can be achieved by: introducing the control solution sample into an electrochemical cell of a sample analyzing device to cause a transformation of the glucose in the control solution sample, the electrochemical cell having a first electrode and a second electrode; determining a temperature of the electrochemical cell; calculating a correction based on the temperature; obtaining a glucose concentration; and determining a corrected concentration of the glucose based on the correction factor such that the corrected concentration of the control solution has less than about 6% of error as compared to a referential specification. A known method of temperature correction for control solutions is given in EP 2,138,841.

These and other embodiments, features and advantages will become apparent to those skilled in the art when taken with reference to the following more detailed description of various exemplary embodiments of the invention in conjunction with the accompanying drawings that are first briefly described.

BRIEF DESCRIPTION OF THE DRAWINGS

The accompanying drawings, which are incorporated herein and constitute part of this specification, illustrate presently preferred embodiments of the invention, and, together with the general description given above and the detailed description given below, serve to explain features of the invention (in which like numerals represent like elements), of which

FIG. 1A illustrates a perspective view of a test strip;

FIG. 1B illustrates an exploded perspective view of the test strip of FIG. 1A;

FIG. 1C illustrates a perspective view of a distal portion of the test strip of FIG. 1A;

FIG. 2 illustrates a bottom plan view of the test strip of FIG. 1A;

FIG. 3 illustrates a side plan view of the test strip of FIG. 1A;

FIG. 4A illustrates a top plan view of the test strip of FIG. 1A;

FIG. 4B illustrates a partial side view of the distal portion of the test strip consistent with arrows 4B-4B of FIG. 4A;

FIG. 5 illustrates a simplified schematic showing a test meter electrically interfacing with the test strip contact pads;

FIG 6 illustrates an exploded view of an immunosensor;

FIG. 7A illustrates a test voltage waveform in which the test meter applies a plurality of test voltages for prescribed time intervals;

FIG. 7B illustrates a test current transient generated with the test voltage waveform of FIG. 7A;

FIG. 8A illustrates a test voltage waveform in which the test meter applies a plurality of test voltages at opposite polarity for prescribed time intervals as compared to Fig. 7A;

FIG. 8B illustrates a test current transient generated with the test voltages of FIG. 8A; and

FIG. 9 is a flow diagram showing the method according to the invention of applying a temperature correction when a sample has been determined to be a control solution.

DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention.

As used herein, the terms "about" or "approximately" for any numerical values or ranges indicate a suitable dimensional tolerance that allows the part or collection of components to function for its intended purpose as described herein. In addition, as used herein, the terms "patient," "host," "user," and "subject" refer to any human or animal subject and are not intended to limit the systems or methods to human use, although use of the subject invention in a human patient is preferred.

Those skilled in the art will understand that the systems and methods specifically described herein and illustrated in the accompanying drawings are non-limiting exemplary embodiments and that the scope of the present disclosure is defined solely by the claims.

Determination of a wide variety of analytes in a wide variety of samples may be achieved, and are particularly suited for use in the determination of analytes in whole blood, plasma, serum, interstitial fluid, or derivatives thereof. A glucose test system may be based on a thin-layer cell design with opposing electrodes and tri-pulse electrochemical detection that is fast (e.g., about 5 second analysis time), requires a small sample (e.g., about 0.4 $\mu$L), and can provide improved reliability and accuracy of blood glucose measurements. In the reaction cell to assay analyte, glucose in the sample can be oxidized to gluconolactone using glucose dehydrogenase and an electrochemically active mediator can be used to shuttle electrons from the enzyme to a palladium working electrode. More particularly, a reagent layer coating at least one of the electrodes in the reaction cell can include glucose dehydrogenase (GDH) based on pyrroloquinoline quinone (PQQ) co-factor and ferricyanide. The enzyme GDH based on the PQQ co-factor may be replaced with the enzyme GDH based on the flavin adenine dinucleotide (FAD) co-factor. When blood or control solution is dosed into the reaction chamber, glucose is oxidized by GDN(ox) and in the process converts GDH(ox) to GDH(red), as shown in the chemical transformation T.1 below. Note that GDH(ox) refers to the oxidized state of GDH, and GDH (red) refers to the reduced state of GDH.

$$\text{T.1 D-Glucose} + \text{GDH}_{(xo)} \rightarrow \text{Gluconic acid} + \text{GDH}_{(red)}$$

Next, GDH(red) is regenerated back to its active oxidized state by ferricyanide (i.e. oxidized mediator or $Fe(CN)_6^{3-}$) as shown in chemical transformation T.2 below. In the process of regenerating GDH(ox), ferrocyanide (i.e. reduced mediator or $Fe(CN)_6^{4-}$) is generated from the reaction as shown in T.2:

$$\text{T.2 GDH}_{(red)} + 2\ Fe(CN)_6^{3-} \rightarrow \text{GDH(ox)} + 2\ Fe(CN)_6^{4-}$$

A potentiostat can be utilized to apply a tri-pulse potential waveform to the working and counter electrodes, resulting in test current transients used to calculate the glucose concentration. Further, additional information gained from the test current transients may be used to discriminate between sample matrices and correct for variability in blood samples due to hematocrit, temperature variation, electrochemically active components, and identify possible system errors.

In principle, any type of electrochemical cell having spaced apart first and second electrodes and a reagent layer may be used. For example, an electrochemical cell can be in the form of a test strip. In one aspect, the test strip may include two opposing electrodes separated by a thin spacer for defining a sample-receiving chamber or zone in which a reagent layer is located. Applicants note that other types of test strips, including, for example, test strips with co-planar electrodes may also be used with the methods described herein.

**Electrochemical Cells**

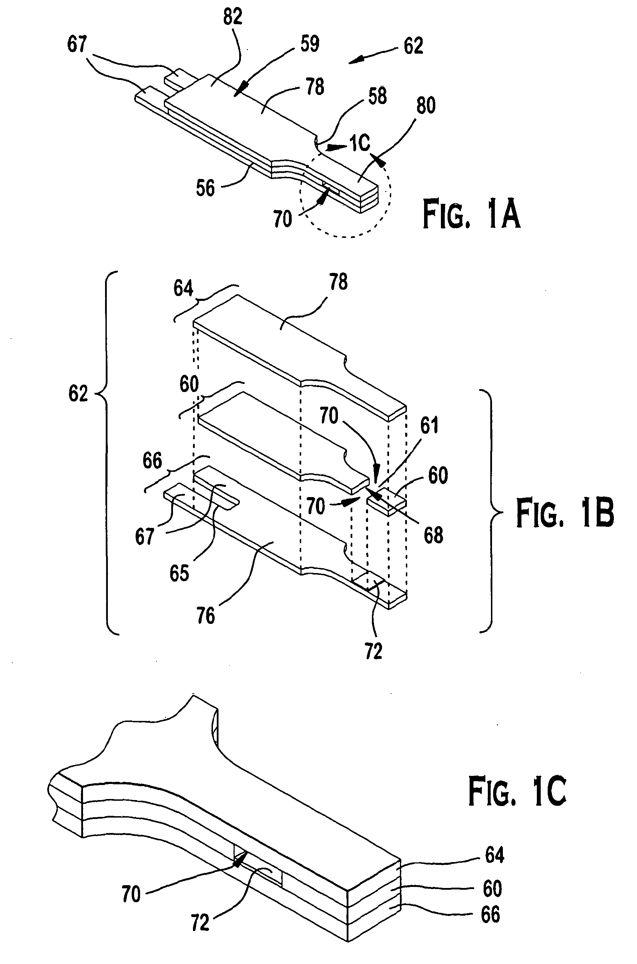

FIGS. 1A-4B show various views of an exemplary test strip 62. As shown, the test strip 62 can include an elongate body extending from a proximal end 80 to a distal end 82, and having lateral edges 56, 58. The proximal portion of the body 59 can include a sample reaction chamber 61 having multiple electrodes 164, 166 and a reagent 72, while the distal portion of the test strip body 59 can include features configured for electrically communicating with a test meter. In use, physiological fluid or a control solution can be delivered to the sample reaction chamber 61 for electrochemical analysis.

The test strip 62 can include a first electrode layer 66 and a second electrode layer 64, with a spacer layer 60 positioned therebetween. The first electrode layer 66 can provide a first electrode 166 and a first connection track 76 for electrically connecting the first electrode 166 to a first electrical contact 67. Similarly, the second electrode layer 64 can provide a second electrode 164 and a second connection track 78 for electrically connecting the second electrode 164 with a second electrical contact 63.

The sample reaction chamber 61 is defined by the first electrode 166, the second electrode 164, and a spacer 60 as shown in FIGS. 1A-4B. Specifically, the first electrode 166 and the second electrode 164 define, respectively, the bottom and top of the sample reaction chamber 61. A cutout area 68 of the spacer 60 can define the side walls of the sample reaction chamber 61. In one aspect, the sample reaction chamber 61 can further include a number of ports 70 that provide a sample inlet and/or a vent. For example, one of the ports can provide a fluid sample ingress and the other port can act as a vent.

The sample reaction chamber 61 can have a small volume. For example, the volume can range from about 0.1 microliters to about 5 microliters, preferably about 0.2 microliters to about 3 microliters, and more preferably about 0.3 microliters to about 1 microliter. As will be appreciated by those skilled in the art, the sample reaction chamber 61 can have various other such volumes. To provide the small sample volume, the cutout 68 can have an area ranging from about 0.01 $cm^2$ to about 0.2 $cm^2$, preferably about 0.02 $cm^2$ to about 0.15 $cm^2$, and more preferably about 0.03 $cm^2$ to about 0.08 $cm^2$. Similarly, those skilled in the art will appreciate that the volume cutout 68 can be of various other such areas. In addition, the first and second electrode 166, 164 can be spaced in the range of about 1 micron to about 500 microns, preferably in the range of about 10 microns to about 400 microns, and more preferably in the range of about 40 microns to about 200 microns. Such a range can vary between various other values. The close spacing of the electrodes can also allow redox cycling to occur, where oxidized mediator generated at the first electrode 166, can diffuse to the second electrode 164 to become reduced, and subsequently diffuse back to the first electrode 166 to become oxidized again.

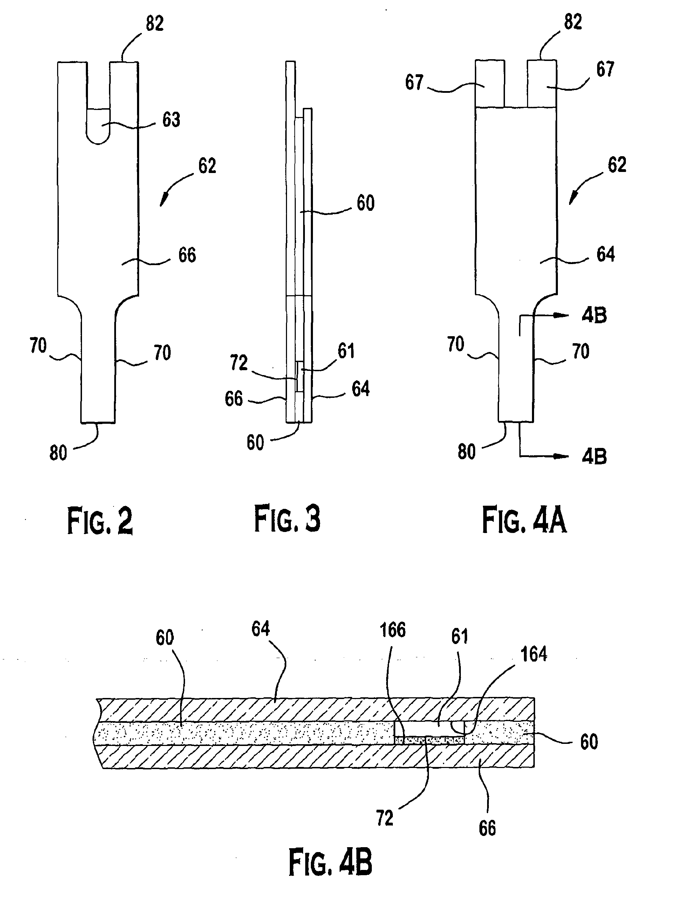

At the distal end of the test strip body 59, a first electrical contact 67 can be used to establish an electrical connection to a test meter. A second electrical contact 63 can be accessed by the test meter through a U-shaped notch 65 as illustrated in FIG. 2. Applicants note that the test strip 62 can include a variety of alternative electrical contact configured for electrically connecting to a test meter. For example, U.S. Patent No. 6,379,513, discloses an electrochemical cell connection means.

The first electrode layer 66 and/or the second electrode layer 64 can be a conductive material formed from materials such as gold, palladium, carbon, silver, platinum, tin oxide, iridium, indium, and combinations thereof (e.g., indium doped tin oxide). In addition, the electrodes can be formed by disposing a conductive material onto an insulating sheet (not shown) by various processes such as, for example, a sputtering, electroless plating, or a screenprinting process. The second electrode layer 64 can be a sputtered gold electrode and the first electrode layer 66 can be a sputtered palladium electrode. Suitable materials that can be employed as the spacing layer 60 include various insulating materials, such as, for example, plastics (e.g., PET, PETG, polyimide, polycarbonate, polystyrene), silicon, ceramic, glass, adhesives, and combinations thereof.

A reagent layer 72 can be disposed within the sample reaction chamber 61 using a process such as slot coating, dispensing from the end of a tube, ink jetting, and screen printing. Such processes are described, for example, in the following U.S. Patent Nos.: 6,749,887; 6,869,411; 6,676,995; and 6,830,934 The reagent layer 72 can include at least a mediator and an enzyme, and can be deposited onto the first electrode 166. Suitable mediators include ferricyanide, ferrocene, ferrocene derivatives; osmium bipyridyl complexes, and quinone derivatives. Examples of suitable enzymes include glucose oxidase, glucose dehydrogenase (GDH) based on pyrroloquinoline quinone (PQQ) co-factor, GDH based on nicotinamide adenine dinucleotide co-factor, and FAD-based GDH [E.C.1.1.99.10]. One exemplary reagent formulation, which would be suitable for making the reagent layer 72, is described in U.S. Patent No. 7,291,256.

Either the first electrode 166 or the second electrode 164 can function as working electrode which oxidizes or reduces a limiting amount of mediator depending on the polarity of the applied test potential of the test meter. For example, if the current limiting species is a reduced mediator, it can be oxidized at the first electrode 166 as long as a sufficiently positive potential was applied with respect to the second electrode 164. In such a situation, the first electrode 166 performs the function of the working electrode and second electrode 164 performs the function of a counter/reference electrode. It should be noted that unless otherwise stated for test strip 62, all potentials applied by test meter 100 will hereinafter be stated with respect to second electrode 164.

Similarly, if a sufficiently negative potential is applied with respect to the second electrode 164, then the reduced mediator can be oxidized at the second electrode 164. In such a situation, the second electrode 164 can perform the function of the working electrode and the first electrode 166 can perform the function of the counter/reference electrode.

Initially, introducing a quantity of the fluid sample of interest into the test strip 62, which includes the first electrode 166, the second electrode 164 and a reagent layer 72. The fluid sample can be whole blood or a derivative or fraction thereof, or a control solution. The fluid sample, e.g., blood, can be dosed into the sample reaction chamber 61 via the port 70. In one aspect, the port 70 and /or the sample reaction chamber 61 can be configured such that capillary action causes the fluid sample to fill the sample reaction chamber 61.

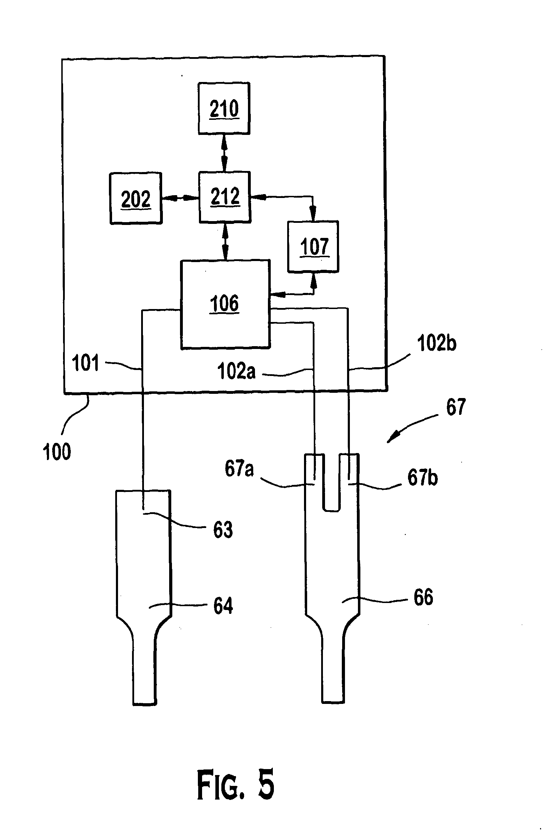

FIG. 5 provides a simplified schematic of a test meter 100 interfacing with a first electrical contact 67 and a second electrical contact 63, which are in electrical communication with the first electrode 166 and the second electrode 164, respectively, of the test strip 62. The test meter 100 can be configured to electrically connect to the first electrode 166 and the second electrode 164 via a first electrical contact 67 and a second electrical contact 63, respectively (as shown in FIGS. 2 and 5). As will be appreciated by those skilled in the art, a variety of test meters can be used with the method described herein. However, the test meter may include at least a processor configured for performing calculations capable of calculating a correction factor in view of at least one measured parameter correlating to a physical property of the electrochemical cell, as well as configured for data sorting and/or storage.

As illustrated in FIG. 5, an electrical contact 67 can include two prongs 67a, 67b. In one exemplary embodiment, the test meter 100 separately connects to the prongs 67a, 67b, such that when the test meter 100 interfaces with a test strip 62 a circuit is completed. The test meter 100 can measure the resistance or electrical continuity between the prongs 67a, 67b to determine whether the test strip 62 is electrically connected to the test meter 100. Applicants note that the test meter 100 can use a variety of sensors and circuits to determine when the test strip 62 is properly positioned with respect to the test meter 100.

The meter 100 can apply a test potential and/or a current between first electrical contact 67 and second electrical contact 63. Once test meter 100 recognizes that strip 62 has been inserted, test meter 100 turns on and initiates a fluid detection mode. In one embodiment, the fluid detection mode causes test meter 100 to apply a constant current of 1 microampere between first electrode 166 and second electrode 164. Because test strip 62 is initially dry, test meter 100 measures a maximum voltage, which is limited by the hardware within test meter 100. However, once a user doses a fluid sample onto inlet 70, this causes sample reaction chamber 61 to become filled. When the fluid sample bridges the gap between first electrode 166 and second electrode 164, test meter 100 will measure a decrease in measured voltage (e.g., as described in U.S. Patent No. 6,193,873), which is below a predetermined threshold causing test meter 100 to automatically initiate the glucose test.

It should be noted that the measured voltage may decrease below a pre-determined threshold when only a fraction of the sample reaction chamber 61 has been filled. A method of automatically recognizing that a fluid was applied does not necessarily indicate that the sample reaction chamber 61 has been completely filled, but can only confirm a presence of some amount of fluid in the sample reaction chamber 61. Once the test meter 100 determines that a fluid has been applied to test strip 62, a short, but non-zero amount of time may still be required to allow the fluid to completely fill the sample reaction chamber 61.

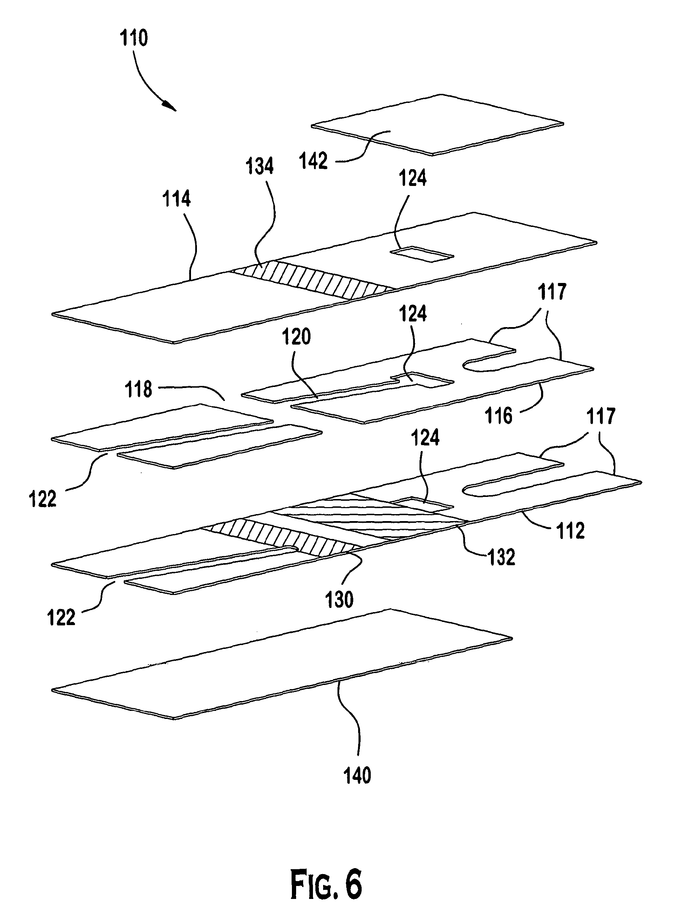

An immunosensor 110, is illustrated in FIG. 6. A plurality of chambers can be formed within the immunosensor, including a fill chamber, by which a sample can be introduced into the immunosensor, a reaction chamber, by which a sample can be reacted with one or more desired materials, and a detection chamber, by which a concentration of a particular component of the sample can be determined. These chambers can be formed in at least a portion of a lower electrode, an upper electrode, and a separator of the immunosensor. The immunosensor can also include a vent hole to allow air to enter and escape the immunosensor as desired, and first and second sealing components to selectively seal first and second sides of the vent hole. The first sealing component can also form a wall of the fill chamber.

As illustrated, the immunosensor 110 includes a lower electrode 112 having two liquid reagents 130, 132 striped onto it. The lower electrode 112 can be formed using any number of techniques used to form electrodes, but in one embodiment a polyethylene tetraphthalate (PET) sheet that is filled with barium sulphate is sputter-coated with gold. Other non-limiting example of forming an electrode are disclosed in U.S. Patent No. 6,521,110 of Hodges et al., entitled "Electrochemical Cell" and filed on November 10, 2000.

Likewise, the liquid reagents 130, 132 can have a number of different compositions. In one embodiment the first liquid reagent 130 includes an antibody conjugated to an enzyme, such as GDH-PQQ, in a buffer that contains sucrose, as well as a poloxamer, such as Pluronics® block copolymers, an anticoagulant, such as citraconate, and calcium ions. The second liquid reagent 132 may include a mixture of ferricyanide, glucose, and a second mediator, such as phenazine ethosulfate, in an acidic buffer, such as a dilute citraconic acid solution. The first and second liquid reagents 130, 132 can be dried onto the lower electrode 112. A number of techniques can be used to dry the reagents 130, 132, but in one embodiment, following the striping of the reagents 130, 132 on the lower electrode 112, one or more infrared dryers can be applied to the reagents 130, 132. One or more air dryers can also be used, for example, subsequent to the infrared dryers. References to a first reagent and a first liquid reagent and a second reagent and a second liquid reagent herein are used interchangeably and are not necessarily an indication that the reagents are in their liquid or dried form at a given time. Further, some of the components associated with the first and second liquid reagents can be used interchangeably and/or in both the first and second liquid reagents as desired. By way of non-limiting example, an anticoagulant can be associated with either or both of the first liquid reagent 130 and the second liquid reagent 132.

A line can be formed in the sputter-coated gold between the reagents 130, 132 such that an edge of reagent 132 is very close to, or touches, the line. The line can be applied using laser ablation or with a sharp metal edge. The line can be applied before the reagents 130, 132 are striped on the electrode. The line can be designed to electrically insulate the section of the lower electrode 112 under the detection chamber from the section that will be under the reaction chamber. This can provide a better definition of an area of the working electrode during the electrochemical assay.

The immunosensor 110 can also include an upper electrode 114 having one or more magnetic beads 134 containing surface-bound antigens thereon. The antigens can be configured to react with the antibody disposed on the lower electrode 112 and the sample within a reaction chamber 118, as described in further detail below. One skilled in the art will recognize that the components disposed on the lower electrode 112 and on the upper electrode 114 can be interchangeable. Thus, the lower electrode 112 can include one or more magnetic beads 134 and the upper electrode 114 can include two liquid reagents 130, 132 striped onto it. Further, although the length of the electrode 112 forms the length of the entire body of the immunosensor 110, in other examples the electrode may only be a portion of a layer of an immunosensor that serves as the lower or upper electrode or multiple electrodes can be disposed on a single layer of an immunosensor. Further, because voltage applied to the immunosensor can be flipped and/or alternated, each of the lower and upper electrodes can serve as the working electrode and the counter or counter/reference electrode at different stages. For ease of description purposes, in the present application the lower electrode is considered the working electrode and the upper electrode the counter or counter/reference electrode.

A separator 116 disposed between the lower and upper electrodes 112, 114 can have a variety of shapes and sizes, but it generally is configured to desirably engage the lower and upper electrodes 112, 114 to form the immunosensor 110. The separator 116 may include adhesive on both sides. The separator 116 can further include a release liner on each side of the two sides of the separator 116. The separator 116 can be cut in a manner that forms at least two cavities. A first cavity can be formed to serve as a reaction chamber 118 and a second cavity can be formed to serve as a detection chamber 120. The separator 116 can be kiss-cut such that the reaction chamber 118 is aligned with the electrodes 112, 114 to allow an antigen-antibody reaction therein while the detection chamber 120 is aligned with the electrodes 112, 114 to allow for the electrochemical determination of ferrocyanide therein.

The separator 116 can be placed on the lower electrode 112 in a manner that allows the magnetic beads 134 of the upper electrode 114 and the first reagent 130 of the lower electrode 112 to be at least partially disposed in the reaction chamber 118 and the ferricyanide-glucose combination of the second reagent 132 of the lower electrode 112 to be at least partially disposed in the detection chamber 120. It can be advantageous to include an anticoagulant in each of the first and second liquid reagents 130, 132 so that an anticoagulant is associated with each of the reaction and detection chambers 118, 120. The combination of one of the upper and lower electrodes 112, 114 and the separator 116 can be laminated together to form a bi-laminate, while in other examples the combination of each of the lower electrode 112, the upper electrode 114, and the separator 116 can be laminated together to form a tri-laminate. Alternatively, additional layers may also be added.

A fill chamber 122 can be formed by punching a hole into one of the lower and upper electrodes 112, 114 and the separator 116. The fill chamber may be formed by punching a hole in the lower electrode 112 and the separator 116 such that the hole in the lower electrode 112 overlaps the reaction chamber 118. As shown, the fill chamber 122 can be a distance apart from the detection chamber 120. Such a configuration allows a sample to enter the immunosensor 110 through the fill chamber 122 and flow into the reaction chamber 118 to be reacted, for example with the first liquid reagent 130 that includes the antibody conjugated to an enzyme in a buffer on the first electrode 112 and the magnetic beads 134 striped on the upper electrode 114, without entering the detection chamber 120. Once the sample has been reacted, it can then flow into the detection chamber 120 to undergo a chemical or physical transformation with the second liquid reagent 132, for example the mixture of ferricyanide, glucose, and the second mediator in an acidic buffer.

A vent 124 can be formed by punching a hole through each of the two electrodes 112, 114 and the separator 116 such that the vent 124 extends through the entirety of the immunosensor 110. The hole can be formed in a suitable manner, such as, for example, drilled or punched in a number of different locations, but it can overlap a region of the detection chamber 120 that is spaced apart from the reaction chamber 118.

The vent 124 can be sealed in a number of different manners. A first sealing component 140 may be located on the lower electrode 112 to seal a first side of the vent 124 and a second sealing component 142 is located on the upper electrode 114 to seal a second side of the vent 124. The sealing components can be made of and/or include any number of materials. By way of non-limiting example, either or both of the sealing components can be hydrophilic adhesive tape or Scotch® tape. Adhesive sides of the sealing components can face the immunosensor 110. As shown, not only can the first sealing component 140 forms a seal for the vent 124, but it can also form a wall for the fill chamber 122 so that the sample can be contained therein. Properties incorporated onto the adhesive side of the first sealing component 140 can be associated with the fill chamber 122. For example, if the first sealing component 140 includes properties making it hydrophilic and/or water soluble, the fill chamber can remain well-wet when a sample is disposed therein. Further, the sealing components 140, 142 can be selectively associated and disassociated with the immunosensor 110 to provide venting and/or sealing for the immunosensor 110 and the components disposed therein as desired.

Adhesives can generally be used in the construction of the immunosensor.

Non-limiting examples of immunosensors include those described in U.S. Patent Application Publication No. 2003/0180814 of Hodges et al., entitled "Direct Immunosensor Assay" and filed on March 21, 2002, U.S. Patent Application Publication No. 2004/0203137 of Hodges et al., entitled "Immunosensor" and filed on April 22, 2004 and U.S. Patent Publication No. 2010/0006452.

The immunosensor 110 can be configured to be placed into a meter that is configured to apply a potential to the electrodes 112, 114 and measure a current that results from the application of the potential. The immunosensor may include one or more tabs 117 for engaging a meter. Other features can also be used to engage the immunosensor 110 with a meter. The meter can include a number of different features. For example, the meter can include a magnet that is configured to maintain certain components of the immunosensor 110 in one chamber while other components flow to the other. The magnet of the meter may be located such that, upon placing the immunosensor 110 in the meter, the magnet is disposed below the reaction chamber 118. This can allow the magnet to assist in holding back any magnetic beads 134, and more particularly any antibody-enzyme conjugate that is bound to the beads 134, from flowing into the detection chamber 120.

An alternate feature of the meter includes a heating element. A heating element can help speed up the reaction rate and help the sample flow through the immunosensor 110 in a desired manner by reducing the viscosity. A heating element can also allow one or more chambers and/or a sample disposed therein to be heated to a predetermined temperature. Heating to a predetermined temperature can help provide accuracy, for example, by diminishing or removing the effects of temperature change as reactions occur.

Further, a piercing instrument can also be associated with the meter. The piercing instrument can be configured to pierce at least one of the first and second sealing components at a desired time so that air can flow out of the vent hole and liquid can flow from the reaction chamber into the detection chamber.

The immunosensor 110 and the test strip 62 can also be configured to be associated with a control unit, which in the preferred embodiment, may be the Texas Instrument MSP-430 microcontroller. The control unit can be configured to perform a variety of functions. The control unit may be capable of measuring a fill time of a sample when it is introduced to the device. The control unit can be configured to determine a haematocrit value of a blood sample. The control unit can be configured to calculate a concentration of an analyte in the sample in view of the fill time. In fact, the control unit can include a number of different features, depending, at least in part, on the functionality desired and the method by which the system is designed to measure the fill time.

The control unit can also measure other aspects of the system. By way of non-limiting example, the control unit can be configured to measure a temperature of one or more chambers of the immunosensor or test strip. It can also be configured to measure a temperature of the sample, a color of the sample, a capacitance of the immunosensor or test strip or a variety of other characteristics and/or properties of the sample and/or the system. By way of further non-limiting example, the control unit can be configured to communicate the results of the fill time determination, the results of the capacitance measurement, the results of the analyte concentration determination, and/or the haematocrit measurement to outside equipment. This can be accomplished in any number of ways. The control unit can be hardwired to a microprocessor and/or a display device. The control unit can be configured to wirelessly transmit data from the control unit to a microprocessor and/or a display device.

Other components of the system can also be configured to make such measurements. For example, the immunosensor or the meter can be configured to measure a temperature of one or more chambers of the immunosensor or test strip, measure or infer the temperature of a sample, or measure, determine, or infer a variety of other characteristics and/or properties of the sample and/or the system. Still further, one skilled in the art will recognize that these features of a control unit can be interchanged and selectively combined in a single control unit. For example, a control unit can both determine a fill time, a capacitance, and measure a temperature of a chamber. Multiple control units can be used together to perform various functions, based at least in part on the configurations of the various control units and the desired functions to be performed.

**Analyte Concentration Test**

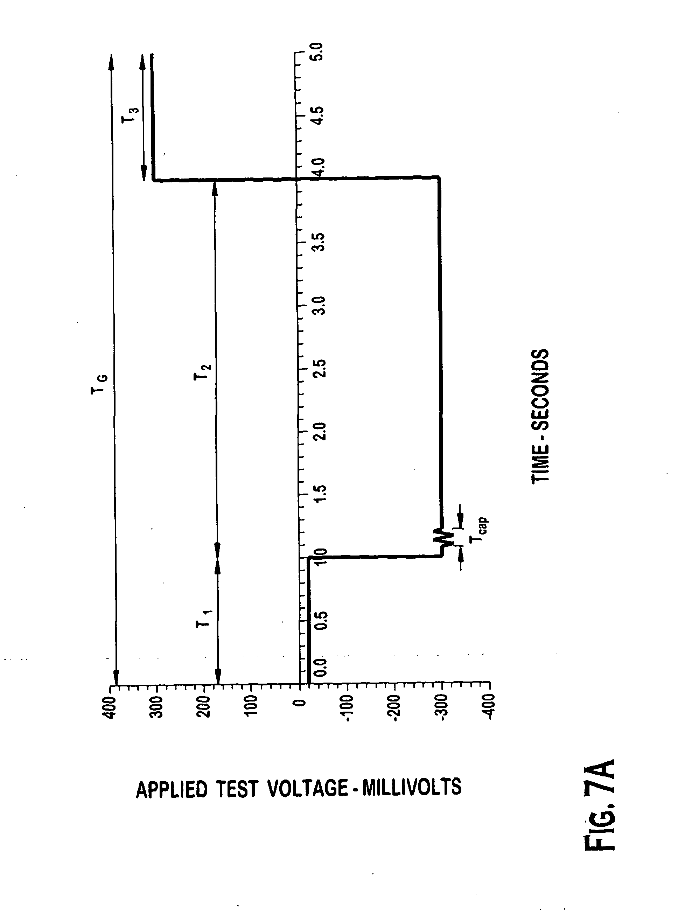

Once the test meter 100 has determined that a fluid has been introduced (e.g., dosed) onto the test strip 62, a test meter 100 can perform a glucose test by applying a plurality of test potentials to the test strip 62 for prescribed intervals as shown in FIG. 7A. A glucose test time interval $T_G$ represents an amount of time to perform the glucose test (but not necessarily all the calculations associated with the glucose test) where the glucose test time interval $T_G$ can include a first test potential $E_1$ for a first test potential time interval $T_1$, a second test potential $E_2$ for a second test potential time interval $T_2$, and a third test potential $E_3$ for a third test potential time interval $T_3$. Further, as illustrated in FIG. 7A, the second test potential time interval $T_2$ can include a constant (DC) test voltage component and a superimposed alternating (AC), or oscillating, test voltage component. The superimposed alternating test voltage component can be applied for a time interval indicated by $T_{cap}$. The glucose test time interval $T_G$ can range, for example, from about 1 second to about 5 seconds.

As discussed above, either the first electrode 166 or the second electrode 164 can function as working electrode which oxidizes or reduces a limiting amount of mediator depending on the polarity of the applied test potential of the test meter. It should be noted that unless otherwise stated all potentials applied by test meter 100 will hereinafter be stated with respect to second electrode 164. However, applicants note that the test potentials applied by test meter 100 can also be stated with respect to the first electrode 166, in which case the polarity of the test potentials and measured currents discussed below would be reversed.

The plurality of test current values measured during the first, second, and third test potential time intervals may be performed at a frequency ranging from about 1 measurement per nanosecond to about one measurement per 100 milliseconds. Applicants note that names "first," "second," and "third" are chosen for convenience and do not necessarily reflect the order in which the test potentials are applied. For instance, a potential waveform where the third test voltage can be applied before the application of the first and second test voltage. Using three test voltages in a serial manner is described, applicants note that the glucose test can include different numbers of open-circuit and test voltages. Applicants also note that the glucose test time interval can include any number open-circuit potential time intervals. For example, the glucose test time interval could include only two test potential time intervals and/or open circuit potential time intervals before and/or after one or more test potential time intervals. The glucose test could include an open-circuit for a first time interval, a second test voltage for a second time interval, and a third test voltage for a third time interval.

As shown in FIG. 7A, the test meter 100 may apply a first test potential $E_1$ (e.g., -20 mV) for a first test potential time interval $T_1$ (e.g., in the range of about 0 to about 1 second). The first test potential time interval $T_1$ can range from about 0.1 seconds to about 3 seconds and preferably range from about 0.2 seconds to about 2 seconds, and most preferably range from about 0.3 seconds to about 1 seconds. The first test potential time interval $T_1$ may be sufficiently long so that the sample reaction chamber 61 can fully fill with sample and also so that the reagent layer 72 can at least partially dissolve or solvate. The first test potential time interval $T_1$ can include any other desired time ranges.

The test meter 100 can apply a first test potential $E_1$ between the electrodes for a duration between when the meter can detect that the strip is filling with sample and before a second test potential $E_2$ is applied. In one aspect, the test potential $E_1$ is small. For example, the potential can be in the range of about -1 to about -100 mV, preferably in the range of about -5 mV to about -50 mV and most preferably in the range of about -10 mV to about -30 mV. The smaller potential perturbs the reduced mediator concentration gradient to a lesser extent compared to applying a larger potential difference, but is still sufficient to obtain a measure of the oxidizable substances in the sample. The test potential $E_1$ can be applied for a portion of the time between detection of fill and when the second test potential $E_2$ is applied or can be applied for the whole of that time period. If the test potential $E_1$ is to be used for a portion of the time then an open-circuit could be applied for the remaining portion of the time. The combination of any number of open-circuit and small voltage potential applications, their order and times applied is not critical, can be applied as long as the total period for which the small potential $E_1$ is applied is sufficient to obtain a current measurement indicative of the presence and/or quantity of oxidizable substances present in the sample. The small potential $E_1$ may be applied for substantially the entire period between when a fill is detected and when the second test potential $E_2$ is applied.

During the first time interval $T_1$, the test meter 100 measures the resulting first current transient, which can be referred to as $i_a(t)$. A current transient represents a plurality of current values measured by a test meter during a particular test potential time interval. A current transient represents a plurality of current values measured by a test meter during a particular test potential time interval. The first current transient $i_a(t)$ can be measured for a time in the range of about 0.05 seconds to about 1.0 second and preferably in the range of about 0.1 seconds to about 0.5 seconds, and most preferably in the range of about 0.1 seconds to about 0.2 seconds. The first current transient $i_a(t)$ can be measured can be measured for any other desired time ranges. As discussed below, a portion or all of the first current transient can be used to determine whether a control solution or a blood sample was applied to the test strip 62. The magnitude of the first transient current is affected by the presence of easily oxidizable substances in the sample. Blood usually contains endogenous and exogenous compounds that are easily oxidized at second electrode 164. Conversely, the control solution can be formulated such that it does not contain oxidizable compounds. However, blood sample composition can vary and the magnitude of the first current transient for high viscosity blood samples will typically be smaller than low viscosity samples (in some cases even less than the control solution samples) because the sample reaction chamber 61 may be not be completely filled after about 0.2 seconds. An incomplete fill will cause the effective area of the first electrode 166 and the second electrode 164 to decrease which in turn can cause the first current transient to decrease. Thus, the presence of oxidizable substances in a sample, by itself, is not always a sufficient discriminatory factor because of variations in blood samples.

Once the first time interval $T_1$ time has elapsed, the test meter 100 can apply a second test potential $E_2$ between the first electrode 166 and the second electrode 164 (e.g., about -300 mV as illustrated in FIG. 7A) for a second test potential time interval $T_2$ (e.g., about 3 seconds as illustrated in FIG. 7A). The second test potential $E_2$ may be a value sufficiently negative of the mediator redox potential so that a limiting oxidation current occurs at the second electrode 164. For example, when using ferricyanide and/or ferrocyanide as the mediator, the second test potential $E_2$ can range from about -600 mV to about zero mV, preferably range from about -600 mV to about -100 mV, and more preferably be about -300 mV. Likewise, the time interval indicated as $T_{cap}$ in FIG. 6 may also last over a range of times, it may have a duration of about 20 milliseconds. The superimposed alternating test voltage component may be appplied after about 0.3 seconds to about 0.32 seconds after the application of the second test voltage $V_2$, and induces two cycles of a sine wave having a frequency of about 109 Hz with an amplitude of about +/-50 mV. During the second test potential time interval $T_2$, the test meter 100 can measure a second current transient $i_b(t)$.

The second test potential time interval $T_2$ may be sufficiently long to monitor the rate of generation of reduced mediator (e.g., ferrocyanide) in the sample reaction chamber 61 based on the magnitude of a limiting oxidation current. The reduced mediator may be generated by a series of chemical reactions in the reagent layer 72. During the second test potential time interval $T_2$, a limiting amount of reduced mediator is oxidized at the second electrode 164 and a non-limiting amount of oxidized mediator is reduced at the first electrode 166 to form a concentration gradient between the first electrode 166 and the second electrode 164. As will be described, the second test potential time interval $T_2$ should be sufficiently long so that a sufficient amount of ferricyanide can be generated at the second electrode 164. A sufficient amount of ferricyanide may be required at the second electrode 164 so that a limiting current can be measured for oxidizing ferrocyanide at the first electrode 166 during the third test potential $E_3$. The second test potential time interval $T_2$ can range from about 0 seconds to about 60 seconds and preferably range from about 1 second to about 10 seconds, and most preferably range from about 2 seconds to about 5 seconds.

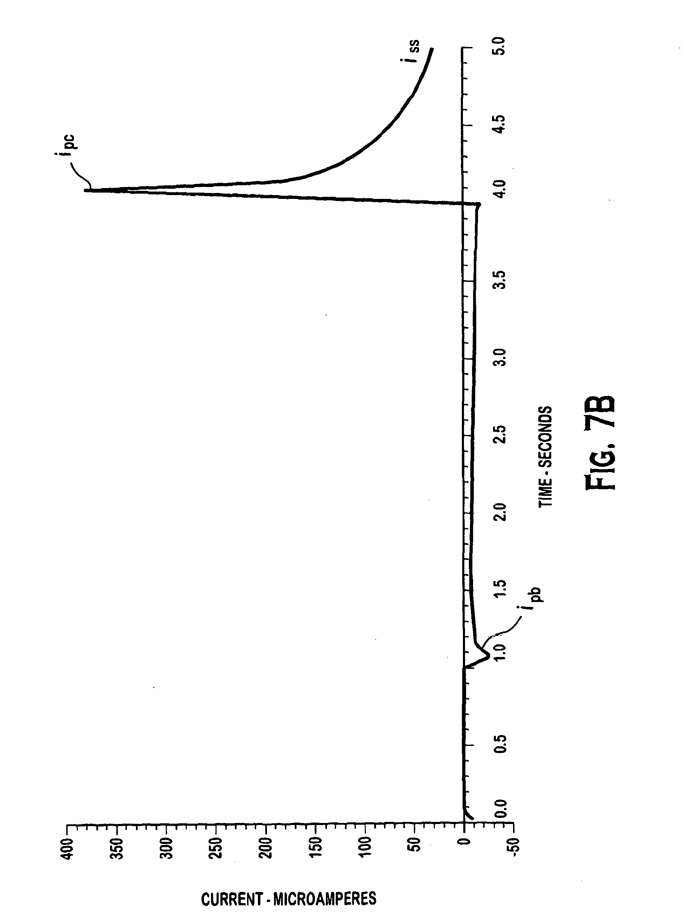

FIG. 7B shows a relatively small peak $i_{pb}$ at the beginning of the second test potential time interval $T_2$ followed by a gradual increase of an absolute value of an oxidation current during the second test potential time interval (e.g., in the range of about 1 second to about 4 seconds). The small peak occurs due to an initial depletion of reduced mediator at about I second. The gradual increase in oxidation current is ascribed to the generation of ferrocyanide by reagent layer 72 followed by its diffusion to the second electrode 164.

After the second potential time interval $T_2$ has elapsed, the test meter 100 can apply a third test potential $E_3$ between the first electrode 166 and the second electrode 164 for a third test potential time interval $T_3$ (e.g., in the range of about 4 to about 5 seconds as illustrated in FIG. 6). During the third test potential time interval $T_3$, the test meter 100 can measure a third current transient, which may be referred to as $i_c(t)$. The third test potential $E_3$ may be a value sufficiently positive of the mediator redox potential so that a limiting oxidation current is measured at the first electrode 166. For example, when using ferricyanide and/or ferrocyanide as the mediator, the third test potential $E_3$ can range from about zero mV to about 600 mV, preferably range from about 100 mV to about 600 mV, and more preferably be about 300 mV.

The second test potential time interval $T_2$ and the third test potential time interval $T_3$ can each range from about 0.1 seconds to about 4 seconds. As shown in FIG. 7A, the second test potential time interval $T_2$ was about 3 seconds and the third test potential time interval $T_3$ was about 1 second. As mentioned above, an open circuit potential time period can be allowed to elapse between the second test potential $E_2$ and the third test potential $E_3$. Alternatively, the third test potential $E_3$ can be applied following the application of the second test potential $E_2$. Note that a portion of the first, second, or third current transient may be generally referred to as a cell current or a current value.

The third test potential time interval $T_3$ may be sufficiently long to monitor the diffusion of a reduced mediator (e.g., ferrocyanide) near the first electrode 166 based on the magnitude of the oxidation current. During the third test potential time interval $T_3$, a limiting amount of reduced mediator is oxidized at the first electrode 166 and a non-limiting amount of oxidized mediator is reduced at the second electrode 164. The third test potential time interval $T_3$ can range from about 0.1 seconds to about 5 seconds and preferably range from about 0.3 seconds to about 3 seconds, and most preferably range from about 0.5 seconds to about 2 seconds.

FIG. 7B shows a relatively large peak $i_{pc}$ at the beginning of the third test potential time interval $T_3$ followed by a decrease to a steady-state current. The first test potential $E_1$ and the second test potential $E_2$ may both have a first polarity, and the third test potential $E_3$ may have a second polarity, which is opposite to the first polarity. However, applicants note that the polarity of the first, second, and third test potentials can be chosen depending on the manner in which analyte concentration is determined and/or depending on the manner in which the test samples and control solutions are distinguished.

**Capacitance Measurement**

A capacitance can be measured, which is essentially an ionic double-layer capacitance resulting from the formation of ionic layers at the electrode-liquid interface. A magnitude of the capacitance can be used to determine whether a sample is control solution or a blood sample. For example, when a control solution is within the reaction chamber, the magnitude of the measured capacitance can be greater than the magnitude of the measured capacitance when a blood sample is in the reaction chamber. As will be discussed in more detail below, a measured capacitance can be used in various methods to correct for the effects of a physical property of the electrochemical cell on measurements made using the electrochemical cell. For example, the measured capacitance can be related to at least one of an age of the electrochemical cell and a storage condition of the electrochemical cell.

By way of non-limiting example, methods and mechanisms for performing capacitance measurements on test strips can be found in U.S. Patents Nos. 7,195,704 and 7,199,594. A test voltage having a constant component and an oscillating component may be applied to the test strip. In such an instance, the resulting test current can be mathematically processed, as described in further detail below, to determine a capacitance value.

Generally, when a limiting test current occurs at a working electrode having a well-defined area (i.e., an area not changing during the capacitance measurement), the most accurate and precise capacitance measurements in an electrochemical test strip can be performed. A well-defined electrode area that does not change with time can occur when there is a tight seal between the electrode and the spacer. The test current is relatively constant when the current is not changing rapidly due either to glucose oxidation or electrochemical decay. Alternatively, any period of time when an increase in signal, which would be seen due to glucose oxidation, is effectively balanced by a decrease in signal, which accompanies electrochemical decay, can also be an appropriate time interval for measuring capacitance.

An area of first electrode 166 can potentially change with time after dosing with the sample if the sample seeps in between the spacer 60 and the first electrode 166. Reagent layer 72 can have an area larger than the cutout area 68 that causes a portion of the reagent layer 72 to be in between the spacer 60 and the first electrode layer 66. Under certain circumstances, interposing a portion of the reagent layer 72 in between the spacer 60 and the first electrode layer 66 can allow the wetted electrode area to increase during a test. As a result, a leakage can occur during a test that causes the area of the first electrode to increase with time, which in turn can distort a capacitance measurement.

In contrast, an area of second electrode 164 can be more stable with time compared to the first electrode 166 because there is no reagent layer in between the second electrode 164 and the spacer 60. Thus, the sample is less likely to seep in between the spacer 60 and the second electrode 164. A capacitance measurement that uses a limiting test current at the second electrode 164 can thus be more precise because the area does not change during the test.

As discussed above and as shown in FIG. 7A, once liquid is detected in the test strip, first test potential $E_1$ (e.g., -20 mV) can be applied between the electrodes for about 1 second to monitor the fill behavior of the liquid and to distinguish between control solution and blood. In Equation 1, the test currents are used from about 0.05 to 1 second. This first test potential $E_1$ can be relatively low such that the distribution offerrocyanide in the cell is disturbed as little as possible by the electrochemical reactions occurring at the first and second electrodes.

A second test potential $E_2$ (e.g., -300 mV) having a larger absolute magnitude can be applied after the first test potential $E_1$ such that a limiting current can be measured at the second electrode 164. The second test potential $E_2$ can include an AC voltage component and a DC voltage component. The AC voltage component can be applied at a predetermined amount of time after the application of the second test potential $E_2$, and further, can be a sine wave having a frequency of about 109 Hertz and an amplitude of about +/-50 millivolts. In a preferred embodiment, the predetermined amount of time can range from about 0.3 seconds to about 0.4 seconds after the application of the second test potential $E_2$. Alternatively, the predetermined amount of time can be a time where a test current transient as a function of time has a slope of about zero. The predetermined amount of time can be a time required for a peak current value (e.g., $i_{pb}$) to decay by about 50%. As for the DC voltage component, it can be applied at a beginning of the first test potential. The DC voltage component can have a magnitude sufficient to cause a limiting test current at the second electrode such as, for example, about -300 mV with respect to the second electrode.

Consistent with FIG. 4B, the reagent layer 72 is not coated onto the second electrode 164, which causes the magnitude of the absolute peak current $i_{pb}$ to be relatively low compared to the magnitude of the absolute peak current $i_{pc}$. The reagent layer 72 can be configured to generate a reduced mediator in a presence of an analyte, and the amount of the reduced mediator proximate to first electrode can contribute to the relatively high absolute peak current $i_{pc}$. At least the enzyme portion of the reagent layer 72 can be configured to not substantially diffuse from the first electrode to the second electrode when a sample is introduced into the test strip.

The test currents after $i_{pb}$ tends to settle to a flat region at approximately 1.3 seconds, and then the current increases again as the reduced mediator generated at the first electrode 166, which can be coated with the reagent layer 72, diffuses to the second electrode 164, which is not coated with the reagent layer 72. A capacitance measurement can be performed at a relatively flat region of the test current values, which can be performed at about 1.3 seconds to about 1.4 seconds. Generally, if the capacitance is measured before 1 second, then the capacitance measurement can interfere with the relatively low first test potential $E_1$ that can be used to measure the first current transient $i_a(t)$. For example, an oscillating voltage component on the order of +/- 50 mV superimposed onto a -20 mV constant voltage component can cause significant perturbation of the measured test current. Not only does the oscillating voltage component interfere with the first test potential $E_1$, but it can also significantly perturb the test currents measured at about 1.1 seconds, which in turn can interfere with correction for antioxidants. Following a great deal of testing and experimentation, it was finally determined that, surprisingly, measuring the capacitance at about 1.3 seconds to about 1.4 seconds resulted in accurate and precise measurements that did not interfere with the control solution/blood discrimination test or the blood glucose algorithm.

After the second test potential $E_2$, third test potential $E_3$ (e.g., +300 mV) can be applied causing the test current to be measured at the first electrode 166, which can be coated with the reagent layer 72. The presence of a reagent layer on the first electrode can allow penetration of liquid between the spacer layer and the electrode layer, which can cause the electrode area to increase.

As illustrated in FIG. 7A, a 109 Hz AC test voltage ($\pm$50 mV peak-to-peak) can be applied for 2 cycles during the time interval $T_{cap}$. The first cycle can be used as a conditioning pulse and the second cycle can be used to determine the capacitance. The capacitance estimate can be obtained by summing the test current over a portion of the alternating current (AC) wave, subtracting the direct current (DC) offset, and normalizing the result using the AC test voltage amplitude and the AC frequency. This calculation provides a measurement of the capacitance of the strip, which is dominated by the strip sample chamber when it is filled with a sample.

The capacitance can be measured by summing the test current over one quarter of the AC wave on either side of the point in time where the input AC voltage crosses the DC offset, i.e. when the AC component of the input voltage is zero (the zero crossing point). A derivation of how this translates to a measurement of the capacitance is described in further detail below. Equation 1 can show the test current magnitude as a function of time during the time interval $T_{cap}$:

$$\text{Eq. 1} \qquad i(t) = i_o + st + I\sin(\omega t + \phi)$$

where the terms $i_o + st$ represent the test current caused by the constant test voltage component. Generally, the DC current component is considered as changing linearly with time (due to the on-going glucose reaction generating ferrocyanide) and is thus represented by a constant $i_o$, which is the DC current at time zero (the zero crossing point), and s, the slope of the DC current change with time. The AC current component is represented by $I\sin(\omega t + \phi)$, where I is the amplitude of the current wave, $\omega$ is its frequency, and $\phi$ is its phase shift relative to the input voltage wave. The term $\omega$ can also be expressed as $2\pi f$, where $f$ is the frequency of the AC wave in Hertz. The term I can also be expressed as shown in Equation 2:

$$\text{Eq. 2} \qquad I = \frac{V}{|Z|}$$

where V is the amplitude of the applied voltage signal and $|Z|$ is the magnitude of the complex impedance. The term $|Z|$ can also be expressed as shown in Equation 22:

$$\text{Eq. 3} \qquad |Z| = \frac{R}{\sqrt{1 + \tan^2 \phi}} = \frac{R}{\sqrt{1 + \omega^2 R^2 C^2}}$$

where R is the real part of the impedance and C is the capacitance.

Equation 1 can be integrated from one quarter wavelength before the zero crossing point to one quarter wavelength after the zero crossing point to yield Equation 4:

$$\text{Eq. 4} \qquad \int_{-1/4f}^{1/4f} i(t) = i_o \left[t\right]_{-1/4f}^{1/4f} + \frac{s}{2}\left[t^2\right]_{-1/4f}^{1/4f} + I \int_{-1/4f}^{1/4f} \sin(\omega t + \phi),$$

which can be simplified to Equation 5:

$$\text{Eq. 5} \qquad \int_{-\frac{1}{4f}}^{\frac{1}{4f}} i(t) = \frac{i_o}{2f} + \frac{I \sin \phi}{\pi f}.$$

By substituting Eq. 2 into Eq. 1, then into Eq. 4, and then rearranging, Equation 6 results:

$$\text{Eq. 6} \qquad C = \frac{1}{2V}\left( \int_{-\frac{1}{4f}}^{\frac{1}{4f}} i(t) - \frac{i_o}{2f} \right).$$

The integral term in Equation 6 can be approximated using a sum of currents shown in an Equation 7:

$$\text{Eq. 7} \qquad \int_{-\frac{1}{4f}}^{\frac{1}{4f}} i(t) \approx \frac{\frac{1}{n}\sum_{k=1}^{n} i_k}{2f}$$

where the test currents $i_k$ are summed from one quarter wavelength before the zero crossing point to one quarter wavelength past the zero crossing point. Substituting Equation 7 into Equation 6 yields Equation 8:

$$\text{Eq. 8} \qquad C = \frac{\frac{1}{n}\sum_{k=1}^{n} i_k - i_o}{4Vf},$$

in which the DC offset current $i_o$ can be obtained by averaging the test current over one full sine cycle around the zero crossing point.

The capacitance measurements can be obtained by summing the currents not around the voltage zero crossing point, but rather around the maximum AC component of the current. Thus, in Equation 7, rather than sum a quarter wavelength on either side of the voltage zero crossing point, the test current can be summed a quarter wavelength around the current maximum. This is tantamount to assuming that the circuit element responding to the AC excitation is a pure capacitor, so $\phi$ is $\pi/2$. Thus, Equation 5 can be reduced to Equation 9:

$$\text{Eq. 9} \qquad \int_{-\frac{1}{4f}}^{\frac{1}{4f}} i(t) = \frac{i_o}{2f} + \frac{I}{\pi f}.$$

This is a reasonable assumption in this case as the uncoated electrode is polarized such that the DC, or real, component of the current flowing is independent of the voltage applied over the range of voltages used in the AC excitation. Accordingly, the real part of the impedance responding to the AC excitation is infinite, implying a pure capacitive element. Equation 9 can then be used with Equation 6 to yield a simplified capacitance equation that does not require an integral approximation. The net result is that capacitance measurements when summing the currents not around the voltage crossing point, but rather around the maximum AC component of the current, were more precise.

#### CS/Blood Discrimination Test

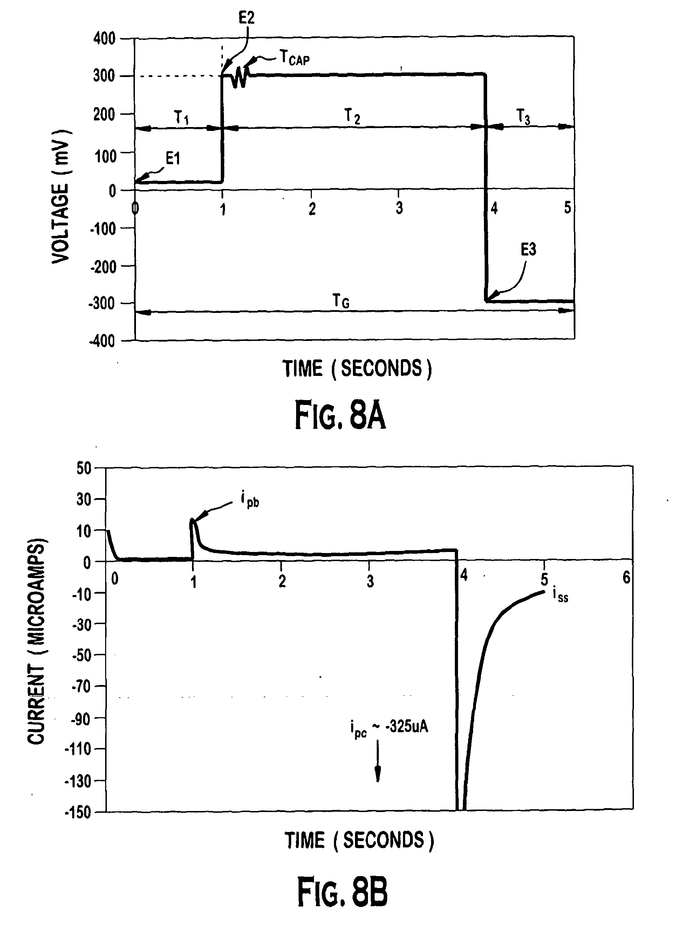

A control solution (CS)/blood discrimination test can be performed. If the CS/blood discrimination test determines that the sample is blood, then a series of steps can be performed that can include: the application of a blood glucose algorithm, hematocrit correction, blood temperature correction, and error checks; and if the CS/blood discrimination test determines that the sample is CS (i.e., not blood), then a series of steps can be performed that can include: the application of a CS glucose algorithm, CS temperature correction, and error checks as shown and described in relation to Figures 8-15 of US Patent Application Publication No. 2009/0301899. If there are no errors, then the test meter outputs a glucose concentration, but if there are errors, then the test can output an error message.

Characteristics of a control solution (CS) may be used to distinguish control solutions from blood. For example, the presence and/or concentration of redox species in the sample, reaction kinetics, and/or capacitance can be used to distinguish control solutions from blood. The method may include the step of calculating a first reference value that is representative of the redox concentration in the sample and a second reference value that is representative of the rate of reaction of the sample with the reagent. The first reference value is an interferent oxidation current and the second reference value is a reaction completion percentage.

A CS/blood discrimination test can include a first reference value and a second reference value. The first value can be calculated based on the current values within the first time interval $T_1$ and the second reference value can be based on current values during both the second time interval $T_2$ and the third time interval $T_3$. The first reference value can be obtained by performing a summation of the current values obtained during the first time current transient when using the test voltage waveform of FIG. 7A. By way of non-limiting example, a first reference value $i_{sum}$ can be represented by Equation 10:

$$\text{Eq. 10} \qquad i_{sum} = \sum_{t=0.05}^{1} i(t)$$

where the term $i_{sum}$ is the summation of current values and t is a time. The second reference value, sometimes referred to as the residual reaction index, can be obtained by a ratio Y of current values during the second time interval and the third time interval, as shown in Eq. 11:

$$\text{Eq. 11} \qquad Y = \text{abs}\left(\frac{i(3.8)}{i(4.15)}\right)$$

where abs represents an absolute value function and 3.8 and 4.15 represent the time in seconds of the second and third time intervals, respectively, for this particular example.

A discrimination criterion can be used to determine if the sample is either control solution or blood based on the first reference value of Eq. 10 and the second reference of Eq. 11. For example, the first reference value of Eq. 10 can be compared to a pre-determined threshold and the second reference value of Eq. 11 can be compared to a pre-determined threshold function. The pre-determined threshold may be, for example, about 12 microamperes. The pre-determined threshold function can be based on a function using the first reference value of Eq. 10. More specifically, as illustrated by Eq. 12, where the calculated value of either of $i_{sum}$ in Eq. 10 is represented *by X,* the pre-determined threshold function $F_{pdt}$ can be:

$$\text{Eq. 12} \qquad F_{PDT} = Z\frac{X-12}{X}$$

where Z can be a constant such as, for example, about 0.2. Thus, the CS/Blood discrimination test can identify a sample as blood if $i_{sum},$ as shown in Eq. 10, is greater than or equal to the predetermined threshold, e.g., about 12 microamperes, and if the ratio Y of current values during the second time interval and the third time interval, as shown in Eq. 11, is less than the value of the pre-determined threshold function $F_{pdt}$, else the sample is a control solution.

**Blood Glucose Algorithm**

If the sample is identified as a blood sample, a blood glucose algorithm can be performed on the test current values. Assuming that a test strip has an opposing face or facing arrangement as shown in FIGS. 1A-4B, and that a potential waveform is applied to the test strip as shown in FIG. 7A or FIG. 8A, a glucose concentration [G] can be calculated using a glucose algorithm as shown in Equation (Eq.) 13:

$$\text{Eq. 13} \qquad [G] = \left(\frac{|i_2|}{|i_3|}\right)^p \left(a|i_1| - Z\right)$$

In Eq. 13, $[G]$ is the glucose concentration, $i_1$ is a first current value, $i_2$ is a second current value, and $i_3$ is a third current value, and the terms $p$, $Z$, and $a$ are empirically derived calibration constants. A derivation of Eq. 13 can be found in a pending U.S. Published Patent Application No. 2007/0074977. All test current values (e.g., $i_1$, $i_2$, and $i_3$) in Equation 13 use the absolute value of the current. The first current value $i_1$ and the second current value $i_2$ are calculated from the third current transient and the third current value $i_3$ is calculated from the second current transient. Applicants note that the names "first," "second," and "third" are chosen for convenience and do not necessarily reflect the order in which the current values are calculated. In addition, all current values (e.g., $i_1$, $i_2$, and $i_3$) stated in Eq. 13 use the absolute value of the current

The term $i_1$ can be defined to include peak current values from the second and third current transients to allow for more accurate glucose concentration as shown in Eq. 14:

$$\text{Eq. 14} \qquad i_1 = i_2 \left\{ \frac{i_{pc} - 2i_{pb} + i_{ss}}{i_{pc} + i_{ss}} \right\}$$

The term $i_{pb}$ represents a peak current value for the second test potential time interval $T_2$ and the term $i_{pc}$ represents a peak current value for the third test potential time interval $T_3$. The term $i_{ss}$ is an estimate of the steady-state current, which is the current predicted to occur at long times after the application of the third test potential $E_3$ in the absence of on-going chemical reactions. Some examples of methods for calculating $i_{ss}$ can be found in U.S. Patent Nos. 5,942,102 and 6,413,410. The use of peak current values to account for interferents in a physiological sample are described in U.S. Published Patent Application No. 2007/0227912.

Eq. 13 and Eq. 14 can be used together to calculate a glucose concentration for either blood or a control solution. The algorithm of Eq. 13 and Eq. 14 can be used for blood with a first set of calibration factors (i.e., $a$, $p$, and Z) and a second set of calibration factors can be used for the control solution. When using two different sets of calibration factors, the methods described herein for discriminating between a test fluid and a control solution can improve the effectiveness of the analyte concentration calculations.

Although the example illustrated in Figs. 7A and 7B shows the polarity of the first and second applied voltages as negative with a third applied voltage as negative, the voltages applied can be of opposite polarity illustrated in Fig. 7A as long as the magnitude and timing of the applied voltages are the same. For example, in Figs. 8A and 8B, the polarity of the first and second applied voltages are positive with the polarity of the third applied voltage as negative. In both cases, the calculation of the glucose is the same because only the absolute values of the sampled resulting current transients are utilized in such calculations.

In addition, if the test meter determines that the sample is control solution (as opposed to blood), the test meter can store the resulting glucose concentration of the control sample such that a user can review test sample concentration data separately from control solution data. For example, the glucose concentrations for control solutions can be stored in a separate database, can be flagged, and/or discarded (i.e., not stored or stored for a short period of time).

Another advantage of being able to recognize a control solution is that a test meter can be programmed to automatically compare the results (e.g., glucose concentration) of the test of the control solution with the expected glucose concentration of the control solution. For example, the test meter can be pre-programmed with the expected glucose level(s) for the control solution(s). Alternatively, a user could input the expected glucose concentration for the control solution. When the test meter recognizes a control solution, the test meter can compare the measured control solution glucose concentration with the expected glucose concentration to determine if the meter is functioning properly. If the measured glucose concentration is out of the expected range, the test meter can output a warning message to alert the user.

### Fill Time Correction

The analyte concentration can be corrected on the basis of the fill time of the sample. A sample may be introduced into an electrochemical cell of a sample analyzing device that has a working electrode and a counter electrode. An electric potential can be applied between the working and counter electrodes of the electrochemical cell and a fill time of the sample into, for example, a capillary space of the electrochemical cell, can be determined. A prepulse time can be calculated in view of at least the fill time of the sample and an electric potential can be applied between the working electrode and the counter electrode for a length of time equal to the prepulse time. A concentration of the analyte in the sample can then be determined. By calculating the prepulse time in view of the fill time, more accurate results can be achieved for analyte concentration. For example, errors, such as those that can result from varying haematocrit levels across samples, can be accounted for, thereby leading to more accurate determinations of the concentrations of the analytes in the samples. In an alternative embodiment for detecting a concentration of an analyte in a sample, errors can be corrected for based on a determined initial fill velocity rather than a determined fill time.

**Temperature Correction**

A blood temperature correction can be applied to the test current values to provide an analyte concentration with an improved accuracy because of a reduced effect from temperature. A method for calculating a temperature corrected analyte concentration can include measuring a temperature value and calculating a temperature correction value $C_T$. The temperature correction value $C_T$ can be based on a temperature value and an analyte concentration, e.g., a glucose concentration. Accordingly, the temperature correction value $C_T$ can then be used to correct the analyte concentration for temperature.

Initially, an analyte concentration uncorrected for temperature can be obtained, such as the glucose concentration [G] from Equation 13, above. A temperature value can also be measured. The temperature can be measured using a thermistor or other temperature reading device that is incorporated into a test meter, or by way of any number of other mechanisms or means. Subsequently, a determination can be performed to determine whether the temperature value T is greater than a first temperature threshold $T_1$. For example, the temperature threshold $T_1$ can be about 15 °C. If the temperature value T is greater than 15 °C, then a first temperature function can be applied to determine the temperature correction value $C_T$. If the temperature value T is not greater than 15 °C, then a second temperature function can be applied to determine the temperature correction value $C_T$.

The first temperature function for calculating the temperature correction value $C_T$ can be in the form of Equation 15:

$$\text{Eq. 15} \qquad C_T = -K_9(T - T_{RT}) + K_{10} \times [G](T - T_{RT})$$

where $C_T$ is the correction value, $K_9$ is a ninth constant (e.g., 0.57), T is a temperature value, $T_{RT}$ is a room temperature value (e.g., 22 °C), $K_{10}$ is a tenth constant (e.g., 0.00023), and [G] is the glucose concentration. When T is about equal to $T_{RT}$, $C_T$ is about zero. In some instances, the first temperature function can be configured to have essentially no correction at room temperature such that variation can be reduced under routine ambient conditions. Another temperature function to calculate another correction value $C_T$ can be in the form of Equation 16:

$$\text{Eq. 16} \quad C_T = -K_{11}(T - T_{RT}) - K_{12} \times [G] \, T - T_{RT}) - K_{13} \times [G](T - T_1) + K_{14} \times [G](T - T_1)$$

where $C_T$ is the correction value, $K_{11}$ is an eleventh constant (e.g., 0.57), T is a temperature value, $T_{RT}$ is a room temperature value, $K_{12}$ is a twelfth constant (e.g., 0.00023), [G] is a glucose concentration, $K_{13}$ is a thirteenth constant (e.g., 0.63), $T_1$ is a first temperature threshold, and $K_{14}$ is a fourteenth constant (e.g., 0.0038).

After $C_T$ is calculated using either Equations 15 or Equation 16, a couple of truncation functions can be performed to ensure that $C_T$ is constrained to a pre-determined range, thereby mitigating the risk of an outlier. $C_T$ can be limited to have a range of -10 to +10. For example, a determination can be performed to determine whether $C_T$ is greater than 10. If $C_T$ is greater than 10, then $C_T$ is set to 10. If $C_T$ is not greater than 10, then a determination is performed to determine whether $C_T$ is less than -10. $C_T$ can be set to -10 if $C_T$ is less than -10. If $C_T$ is a value already in between -10 and +10, then there generally is no need for truncation.

Once $C_T$ is determined, a temperature corrected glucose concentration can be calculated. For example, a determination can be performed to determine whether the glucose concentration uncorrected for temperature (e.g., [G]) is less than 100 mg/dL. If [G] is less than 100 mg/dL, then an Equation 17 can be used to calculate the temperature corrected glucose concentration $G_T$ by adding the correction value $C_T$ to the glucose concentration [G]:

$$\text{Eq. 17} \qquad G_T = [G] + C_T.$$

If [G] is not less than 100 mg/dL, then an Equation 18 can be used to calculate the temperature corrected glucose concentration $G_T$ by dividing $C_T$ by one hundred, adding one; and then multiplying by the glucose concentration [G]:

$$\text{Eq. 18} \qquad G_T = [G]*[1 + 0.01 \times C_T].$$

Once a glucose concentration is determined that has been corrected for the effects of temperature, the glucose concentration can be output, e.g., to a display

According to the present invention, a control solution (CS) temperature correction $C_T$ is applied to the test current value that is different than the blood glucose temperature correction. Once a meter identifies that a control solution has been applied to the test strip, the meter can apply the appropriate type of temperature correction. The control solution temperature correction can provide an analyte concentration with an improved accuracy because of a reduced effect from temperature.

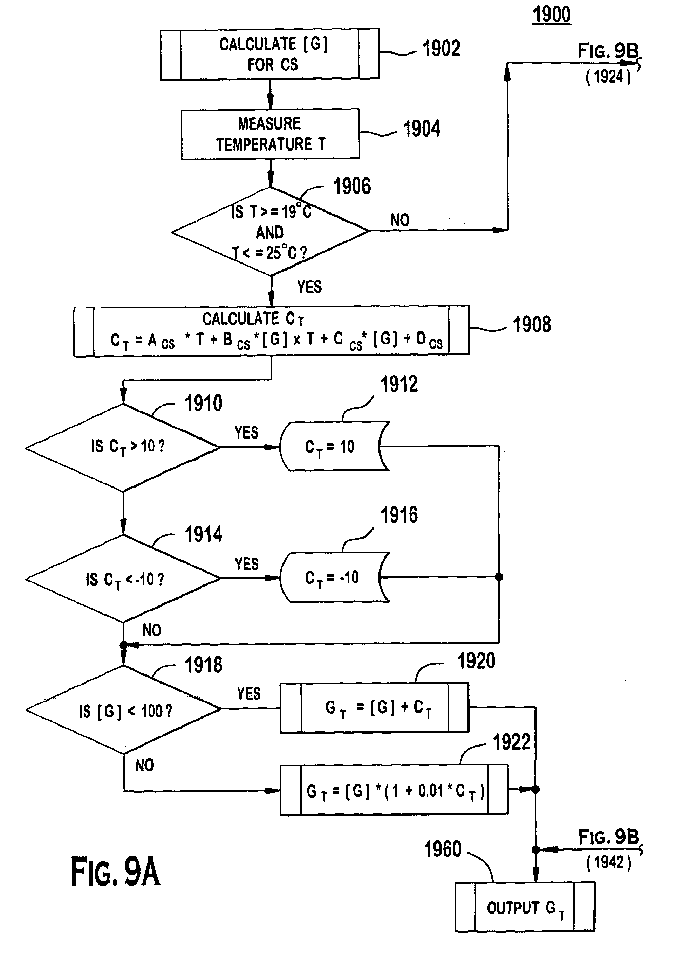

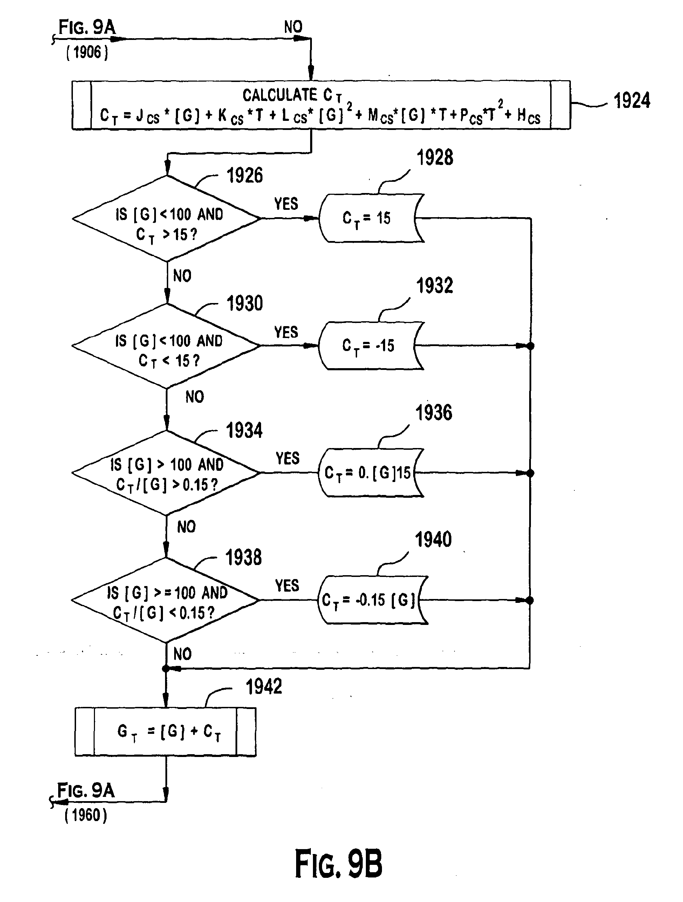

FIG. 9 illustrates the method 1900 according to the invention to determine a glucose concentration of a control solution ("CS") step 1902, which values are corrected for temperature may include a first and second temperature function. A first temperature function may be used when the ambient temperature is within a predetermined range. A second temperature function may be used when the ambient temperature is outside the predetermined range. For example, the predetermined range may range from about 19° C to about 25° C (i.e., $19° C \leq T \leq 25° C$) at step 1906. As shown in step 1906, if the measured temperature T in step 1904 is within the predetermined range, the first temperature function is utilized. The first temperature can be in the form of Equation 16A and calculated in step 1908 of Fig. 9.

$$\text{Eq. 16A} \qquad C_T = A_{CS} \times T + B_{CS} \times [G] \times T + C_{CS} \times [G] + D_{CS}$$

The terms are empirically derived constants, which in this example, $A_{CS} \sim (-0.47$, $B_{CS} \sim (-0.002)$, $C_{CS} \sim 0.04)$ and $D_{CS} \sim 10.3$. Once $C_T$ is determined is using Eq. 16A, a temperature corrected glucose concentration for CS can be calculated using steps 1910, 1912, 1914, 1916, 1918, 1920, 1922, and 1960, as illustrated in FIG. 9. In particular, where the correction factor $C_T$ is calculated in step 1908 as being greater than 10 in step 1910 then such correction factor is set to 10 in step 1912. On the other hand, if the $C_T$ is less than -10 in step 1914 then $C_T$ is set to equal to -10 in step 1916. Where $C_T$ is determined to be within -10 to 10 then the glucose concentration [G] is determined as to whether such concentration is less than 100 mg/dLor greater/equal than 100 mg/dLin step 1918. If the glucose concentration [G] is greater than or equal to 100 mg/dLin step 1918, the temperature-corrected glucose concentration $G_T$ is calculated in step 1920 using Eq. 17 otherwise the temperature-corrected glucose concentration $G_T$ is calculated in step 1922 and the result from either 1920 or 1922 provided in step 1960 to the processor of the meter.

Where the ambient temperature is outside the predetermined range (i.e., $T \leq 19° C$ or $T \geq 25° C$), as determined in step 1906, the second temperature function can be used in step 1924, which is in the form of Equation 16B.

$$\text{Eq. 16B} \qquad C_T = J_{CS}*[G] + K_{CS}*T + L_{CS}*[G]^2 + M_{CS}*[G]*T + P_{CS}*T^2 + H_{CS}$$

The terms Jcs, $K_{CS}$, Lcs, Mcs, $P_{CS}$, and Hcs are empirically derived constants (which in this example are as follows: $H_{CS} \sim 12.6$; $J_{CS} \sim 0.23$; $K_{CS} \sim (-1.7)$; $L_{CS} \sim (-0.0002)$; $M_{CS} \sim (-0.006)$; and $P_{CS} \sim 0.03$). After $C_T$ is calculated using Equation 16B, a truncation subroutine (steps 1926, 1928, 1930, 1932, 1934, 1936, 1938, 1940, 1942) can be performed to ensure that $C_T$ is constrained to a pre-determined range, thereby mitigating the risk of an outlier. In one embodiment where [G] less than about 100 mg/dL, $C_T$ can be limited to have a range of -15 to +15. For example, a determination can be performed to determine whether $C_T$ is greater than 15. If $C_T$ is greater than 15, then $C_T$ is set to 15 (steps 1926, 1928). In step 1926, if $C_T$ is not greater than 15, then a determination is performed to determine whether [G] is less than 100 mg/dLand $C_T$ is less than -15. $C_T$ can be set to -15 if both [G] is less than 100 mg/dLand $C_T$ is less than - 15. If $C_T$ is a value already in between from about -15 to about +15, and [G] less than about 100 mg/dL, then there generally is no need for truncation. However, where [G] is not less than about 100 mg/dL, i.e., [G] is equal to or greater than 100 mg/dL, then Equations 16C and 16D are applied.

$$\text{Eq. 16C} \qquad \text{If } \{C_T / [G]\} > 0.15, \text{ then } C_T = 0.15 \times [G]$$

$$\text{Eq. 16D} \qquad \text{If } \{C_T / [G]\} < -0.15, \text{ then } C_T = -0.15 \times [G]$$

Once $C_T$ is determined using Eqs. 16B, 16C, and 16D with the proper truncation applied, a temperature corrected glucose concentration for CS can be calculated using Eq. 17 in step 1942 with the output of such glucose concentration for the control solution provided in step 1960.

EXAMPLE 1

The following will show an example ofCS measurements using a method of temperature correction described in relation to Figure 9. CS samples were tested at three glucose levels, which were 50 mg/dL, 120 mg/dL, and 350 mg/dL. Three different batch lots of test strips were tested. For each strip lot, about 23 to 70 strips were tested at each CS glucose level at a wide range of temperature and humidity levels. Combinations of temperature and humidity conditions that were tested are 5° C/20% relative humidity (RH), 10° C/70% RH, 22° C/10% RH, 22° C/50% RH, 22° C/90% RH, 35° C/70% RH, and 45° C/10% RH.

Approximately 2,916 sensors were tested to measure a plurality of time CS glucose concentrations using various strip lots, glucose, temperature, and humidity levels. Table 1 below showed that using Eq.'s 16A and 16B resulted in > 95% of tested strips having measured glucose concentration being within specification. Here, the product specification required that the measured CS glucose levels at 50 mg/dL need to be within ±12 mg/dL of the nominal value (which in this case is 50 mg/dL). Similarly, the product specification required that the measured CS glucose levels at the 120 and 350 mg/dL need to be within ±15% of the nominal value (which in this case is 120 and 350 mg/dL, respectively). As can be seen, only one batch has less than 95% of the batch meeting referential specification. In other words, the error is less than 6% and particularly of no more than about 5% error.

**Table 1**

| Target Temperature (°C) | Relative Humidity (%) | Batch | % meeting referential specification |
|---|---|---|---|
| 5 | 20 | 1 | **100** |
| 5 | 20 | 2 | **100** |
| 5 | 20 | 3 | **100** |
| 10 | 70 | 1 | **100** |
| 10 | 70 | 2 | **100** |
| 10 | 70 | 3 | **100** |
| 22 | 10 | 1 | **100** |
| 22 | 10 | 2 | **100** |
| 22 | 10 | 3 | **98.6** |
| 22 | 50 | 1 | **100** |
| 22 | 50 | 2 | **99.5** |
| 22 | 50 | 3 | **100** |
| 22 | 90 | 1 | **100** |
| 22 | 90 | 2 | **97.2** |
| 22 | 90 | 3 | **100** |
| 35 | 70 | 1 | **100** |
| 35 | 70 | 2 | **100** |
| 35 | 70 | 3 | **100** |
| 45 | 10 | 1 | **94.8** |
| 45 | 10 | 2 | **96** |
| 45 | 10 | 3 | **96** |

In addition, Table 2 shows that the results of Table 1 were reproducible.

Table 2

| Target Temperature (°C) | Relative Humidity (%) | Batch | % meeting referential specification |
|---|---|---|---|
| 5 | 20 | 1 | 99.4 |
| 5 | 20 | 2 | 100 |
| 5 | 20 | 3 | 100 |
| 10 | 70 | 1 | 100 |
| 10 | 70 | 2 | 100 |
| 10 | 70 | 3 | 100 |
| 22 | 10 | 1 | 99.4 |
| 22 | 10 | 2 | 99.4 |
| 22 | 10 | 3 | 100 |
| 22 | 50 | 1 | 100 |
| 22 | 50 | 2 | 100 |
| 22 | 50 | 3 | 100 |
| 22 | 90 | 1 | 100 |
| 22 | 90 | 2 | 100 |
| 22 | 90 | 3 | 100 |
| 35 | 70 | 1 | 100 |
| 35 | 70 | 2 | 100 |
| 35 | 70 | 3 | 100 |
| 45 | 10 | 1 | 99.4 |
| 45 | 10 | 2 | 100 |
| 45 | 10 | 3 | 100 |

## EXAMPLE 2

The following provides an embodiment for the preparation of control solution. This preparation is non-limiting as various other preparations and/or control solutions can be utilized with the currently disclosed system and method. Control solution included Citraconic acid Buffer Component at about 0.08 g, Dipotassium citraconate Buffer Component at about 1.9 g, Methyl Paraben Preservative at about 0.05 g, Germal II Preservative at about 0.40 g, Dextran T-500 Viscosity Modifier at about 3.0 g, Pluronic 25R2 Wicking Agent at about 0.05 g, 1-[(6-methoxy-4-sulfo-m-tolyl)azo]-2-naphthol-6-sulfonic acid disodium salt Dye (FD&C Blue No. 1) at about 0.10 g, D-Glucose Analyte at about 50, 120, or 525 mg, and Deionized Water Solvent at about 100 g.

First citraconic buffer pH at about 6.5±0.1 was prepared by dissolving required quantities of citraconic acid and dipotassium citraconate in deionized water. Next, methyl paraben was added and the solution was stirred until the preservative was fully dissolved. Subsequently Dextran T-500, Germal II, Pluronic 25R2 and 1-[(6-methoxy-4-sulfo-m-tolyl)azo]-2-naphthol-6-sulfonic acid disodium salt were added sequentially, following complete dissolution of the previously added chemical. At this point, the pH of the control fluid was verified, followed by addition of the requisite quantity of glucose to obtain a low, normal or high glucose level of the control fluid. After the glucose was dissolved completely, the control fluid was left at room temperature overnight. Finally, the glucose concentration was verified using a Model 2700 Select Biochemistry Analyzer manufactured by Yellow Springs Instrument Co., Inc. The dye used in this control solution has a blue color, which reduces the possibility of a user confusing the control solution with blood.

## Claims

1. A method for determining a concentration of glucose in a control solution sample, the method comprising:

   introducing the control solution sample into an electrochemical cell of a sample analyzing device to cause a transformation of the glucose in the control solution sample, the electrochemical cell having a first electrode and a second electrode;
   determining a temperature of the electrochemical cell;
   calculating a correction based on the temperature;
   obtaining a glucose concentration; and
   determining a corrected concentration of the glucose based on the correction factor,

   wherein the calculating comprises evaluating whether the determined temperature is within a first range and if true the correction factor is calculated with the following equation: $C_T = A_{CS} \times T + B_{CS} \times [G] \times T + C_{CS} \times [G] + D_{CS}$, where [G] comprises the glucose concentration, T comprises determined temperature and the terms $A_{CS}$, $B_{CS}$, $C_{CS}$, and $D_{CS}$ comprise empirically derived constants, and
   wherein the first range comprises a temperature from 19 degrees Celsius to 25 degrees Celsius.

2. The method of claim 1, in which the calculating comprises evaluating whether the determined temperature is within a first range and if false, calculating the correction factor with the following equation: $C_T = J_{CS}*[G] + K_{CS}*T + L_{CS}*[G]^2 + M_{CS}*[G]*T + P_{CS}*T^2 + H_{CS}$, and the terms $J_{CS}$, $K_{CS}$, $L_{CS}$, $M_{CS}$, $P_{CS}$, and $H_{CS}$ comprise empirically derived constants.

3. The method of claim 2, in which the correction factor is limited to a range of from about -15 to bout+15.

4. The method of claim 2, in evaluating comprises determining whether the correction factor is greater than 15 and if true then the correction factor set to a value of about 15 else if the correction factor is not greater than 15, then determining whether the glucose concentration is less than about 100 mg/dL and the correction factor is less than -15.

5. The method of claim 4, in which $C_T$ can be set to -15 if both [G] is less than 100 mg/dL and $C_T$ is less than -15.

6. The method of claim 4, further comprising a determining of whether both conditions of (a) the correction factor comprises a value from about -15 to about +15 and (b) the glucose concentration comprises a value equal to or greater than 100 mg/dL are satisfied, and if true, a value resulting from a division of the correction factor into the glucose concentration is greater than about 0.15 then the correction factor is set as a value equal to about the product of 0.15 times the glucose concentration else if such value is less than -0.15 then the correction factor is set to equal approximately the product of -0.15 times the glucose concentration.

## Patentansprüche

1. Verfahren zum Bestimmen der Konzentration von Glucose in einer Kontrolllösungsprobe, wobei das Verfahren umfasst:

   Einführen der Kontrolllösungsprobe in eine elektrochemische Zelle einer Probenanalyseeinheit, um eine Umwandlung der Glucose in der Kontrolllösungsprobe zu bewirken, wobei die elektrochemische Zelle eine erste Elektrode und eine zweite Elektrode aufweist;
   Bestimmen der Temperatur der elektrochemischen Zelle;
   Berechnen einer Korrektur auf der Grundlage der Temperatur;
   Erhalten einer Glucosekonzentration; und
   Bestimmen einer korrigierten Konzentration der Glucose auf der Grundlage des Korrekturfaktors, wobei die Berechnung das Bestimmen davon umfasst, ob die bestimmte Temperatur in einem ersten Bereich liegt und, wenn zutreffend, der Korrekturfaktor nach folgender Gleichung berechnet wird: $C_T = A_{CS} \times T + B_{CS} \times [G] \times T + C_{CS} \times [G] + D_{CS}$, wobei [G] die Glucosekonzentration umfasst, T die bestimmte Temperatur umfasst und die Begriffe $A_{CS}$, $B_{CS}$, $C_{CS}$ und $D_{CS}$ empirisch abgeleitete Konstanten umfassen, und
   wobei der erste Bereich eine Temperatur von 19 Grad Celsius bis 25 Grad Celsius umfasst.

2. Verfahren gemäß Anspruch 1, wobei die Berechnung das Bestimmen davon umfasst, ob die bestimmte Temperatur in einem ersten Bereich liegt und, wenn nicht zutreffend, der Korrekturfaktor nach folgender Gleichung berechnet wird: $C_T = J_{CS}*[G] + K_{CS}*T + L_{CS}*[G]^2 + M_{CS}*[G]*T + P_{CS}*T^2 + H_{CS}$, und die Begriffe $J_{CS}$, $K_{CS}$, $L_{CS}$, $M_{CS}$, Pcs und $H_{CS}$ empirisch abgeleitete Konstanten umfassen.

3. Verfahren gemäß Anspruch 2, wobei der Korrekturfaktor auf einen Bereich von etwa -15 bis etwa +15 beschränkt ist.

4. Verfahren gemäß Anspruch 2, wobei das Auswerten das Bestimmen davon umfasst, ob der Korrekturfaktor größer als 15 ist, und wenn zutreffend, der Korrekturfaktor auf einen Wert von etwa 15 gesetzt wird, und wenn der Korrekturfaktor nicht größer als 15 ist, Bestimmen, ob die Glucosekonzentration weniger als etwa 100 mg/dl beträgt und der Korrekturfaktor kleiner als -15 ist.

5. Verfahren gemäß Anspruch 4, wobei $C_T$ auf -15 gesetzt werden kann, wenn sowohl [G] kleiner als 100 mg/dl ist als auch $C_T$ kleiner als -15 ist.

6. Verfahren gemäß Anspruch 4, ferner umfassend das Bestimmen davon, ob die beiden Bedingungen (a) der Korrekturfaktor umfasst einen Wert von etwa -15 bis etwa +15 und (b) die Glucosekonzentration umfasst einen Wert von gleich oder größer als 100 mg/dl erfüllt sind, und wenn zutreffend, wenn ein Wert, erhalten durch Teilen des Korrekturfaktors durch die Glucosekonzentration, größer als etwa 0,15 ist, der Korrekturfaktor auf einen Wert von etwa dem Produkt von 0,15 mal der Glucosekonzentration gesetzt wird, und wenn der Wert kleiner als -0,15 ist, der Korrekturfaktor auf etwa das Produkt von -0,15 mal der Glucosekonzentration gesetzt wird.

## Revendications

1. Procédé pour déterminer une concentration de glucose dans un échantillon de solution témoin, le procédé comprenant les étapes suivantes :

   introduire l'échantillon de solution témoin dans une cellule électrochimique d'un dispositif d'analyse d'échantillon pour entrainer une transformation du glucose dans l'échantillon de solution témoin, la cellule électrochimique disposant d'une première électrode et d'une seconde électrode ;
   déterminer une température de la cellule électrochimique ;
   calculer une correction sur la base de la température ;
   obtenir une concentration de glucose ; et
   déterminer une concentration corrigée du glucose sur la base du facteur de correction, où le calcul comprend d'évaluer si la température déterminée se situe dans une première plage et, dans l'affirmative, le facteur de correction est calculé avec l'équation suivante : $C_T = A_{CS}$ x T + $B_{CS}$ x [G] x T + $C_{CS}$ x [G] + $D_{CS}$, où [G] comprend la concentration de glucose, T comprend la température déterminée et les termes $A_{CS}$, $B_{CS}$, $C_{CS}$, et $D_{CS}$ comprennent des constantes dérivées empiriquement,

   et
   où la première plage comprend une température allant de 19 degrés Celsius à 25 degrés Celsius.

2. Procédé selon la revendication 1, dans lequel le calcul comprend d'évaluer si la température déterminée se situe dans la première plage, et dans le cas contraire, calculer le facteur de correction avec l'équation suivante :

$$C_T = J_{CS} * [G] + K_{CS} * T + L_{CS} * [G]^2 + M_{CS} * [G] * T + P_{CS} * T^2 + H_{CS},$$

et les termes $J_{CS}$, $K_{CS}$, $L_{CS}$, $M_{CS}$, Pcs et $H_{CS}$ comprennent des constantes dérivées empiriquement.

3. Procédé selon la revendication 2, dans lequel le facteur de correction est limité à une plage allant d'environ -15 à environ +15.

4. Procédé selon la revendication 2, dans lequel l'évaluation comprend de déterminer si le facteur de correction est supérieur à 15 et, dans l'affirmative, le facteur de correction est alors fixé à une valeur d'environ 15, autrement, si le facteur de correction n'est pas supérieur à 15, alors l'évaluation comprend de déterminer si la concentration de glucose est inférieure à environ 100 mg/dL et le facteur de correction est inférieur à -15.

**5.** Procédé selon la revendication 4, dans lequel $C_T$ peut être fixé à -15 si à la fois [G] est inférieur à 100 mg/dL et $C_T$ est inférieur à -15.

**6.** Procédé selon la revendication 4, comprenant en outre une détermination de savoir si, à la fois, les conditions suivantes : (a) le facteur comprend une valeur allant d'environ -15 à environ +15 et (b) la concentration de glucose comprend une valeur égale à, ou supérieure à, 100 mg/dL, sont satisfaites, et, dans l'affirmative où une valeur résultant d'une division du facteur de correction en la concentration de glucose est supérieure à environ 0,15, le facteur de correction est alors fixé comme valeur égale à environ le produit de 0,15 fois la concentration de glucose, autrement, si une telle valeur est inférieure à -0, 15, le facteur de correction est alors fixé pour égaler approximativement le produit de -0,15 fois la concentration de glucose.

FIG. 1A

FIG. 1B

FIG. 1C

FIG. 2

FIG. 3

FIG. 4A

FIG. 4B

FIG. 5

FIG. 6

FIG. 7A

EP 2 601 520 B1

FIG. 7B

FIG. 8A

FIG. 8B

1900

CALCULATE [G]
FOR CS — 1902

FIG. 9B
(1924)

MEASURE
TEMPERATURE T — 1904

1906

IS T >= 19°C
AND
T <= 25°C ?

NO

YES

CALCULATE $C_T$
$C_T = A_{cs} * T + B_{cs} * [G] \times T + C_{cs} * [G] + D_{cs}$ — 1908

1910

1912

IS $C_T$ > 10 ?

YES

$C_T = 10$

1914

1916

IS $C_T$ < -10 ?

YES

$C_T = -10$

NO

1918

1920

IS [G] < 100 ?

YES

$G_T = [G] + C_T$

NO

1922

$G_T = [G] * (1 + 0.01 * C_T)$

FIG. 9A

FIG. 9B
(1942)

1960

OUTPUT $G_T$

FIG. 9A
(1906)

NO

CALCULATE $C_T$
$C_T = J_{cs} * [G] + K_{cs} * T + L_{cs} * [G]^2 + M_{cs} * [G] * T + P_{cs} * T^2 + H_{cs}$

1924

1926

IS $[G] < 100$ AND $C_T > 15$?

YES

1928

$C_T = 15$

NO

1930

IS $[G] < 100$ AND $C_T < 15$?

YES

1932

$C_T = -15$

NO

1934

IS $[G] > 100$ AND $C_T / [G] > 0.15$?

YES

1936

$C_T = 0.[G]15$

NO

1938

IS $[G] >= 100$ AND $C_T / [G] < 0.15$?

YES

1940

$C_T = -0.15 [G]$

NO

1942

$G_T = [G] + C_T$

FIG. 9A
(1960)

FIG. 9B

30

**EP 2 601 520 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2138841 A
- US 6379513 B
- US 6749887 B
- US 6869411 B
- US 6676995 B
- US 6830934 B
- US 7291256 B
- US 6193873 B
- US 6521110 B, Hodges
- US 20030180814 A, Hodges
- US 20040203137 A, Hodges
- US 20100006452 A
- US 7195704 B
- US 7199594 B
- US 20090301899 A
- US 20070074977 A
- US 5942102 A
- US 6413410 A
- US 20070227912 A